# EUROPEAN PATENT SPECIFICATION

(11) **EP 4 022 087 B1**
(45) Date of publication and mention of the grant of the patent: **31.12.2025**
(21) Application number: 19943483.8
(22) Date of filing: 29.08.2019
(51) Int. Cl.: C12Q 1/686

(54) **EMULSION COMPOSITION FOR DIGITAL PCR AND UNIFORM PARTITIONING METHOD OF PCR SAMPLES THEREFOR**
EMULSIONSZUSAMMENSETZUNG FÜR DIGITALE PCR UND EINHEITLICHES PARTITIONIERUNGSVERFAHREN VON PCR-PROBEN DAFÜR
COMPOSITION D'ÉMULSION POUR PCR NUMÉRIQUE ET PROCÉDÉ DE PARTITIONNEMENT UNIFORME D'ÉCHANTILLONS PCR ASSOCIÉ

(43) Date of publication of application: 06.07.2022
(73) Proprietor: Revosketch Inc., Daejeon 34015 (KR)
(72) Inventor: LEE, Sung Woon, Daejeon 34164 (KR); NAM, Ho Chul, Daejeon 34127 (KR); YOON, Tae Ho, Daejeon 35216 (KR)
(74) Representative: Kraus & Lederer PartGmbB
(86) International application number: PCT/KR2019/011075
(87) International publication number: WO 2021/040093

(56) References cited:
- WO-A1-2015/074898
- WO-A1-2017/209906
- WO-A1-2017/209906
- WO-A1-2018/194240
- WO-A1-2018/194240
- US-A1- 2008 241 841
- US-A1- 2017 275 693
- PHENIX-LAN QUAN ET AL: "dPCR: A Technology Review", SENSORS, vol. 18, no. 4, 20 April 2018 (2018-04-20), pages 1271, XP055624973, DOI: 10.3390/s18041271

## Description

### Technical Field

The present invention relates to an emulsion composition for digital PCR and uniform partitioning method of PCR samples for a digital polymerase chain reaction (digital PCR).

### Background Art

A digital polymerase chain reaction (dPCR) newly emerging in the 21st century, which is a new approach to detecting and quantifying nucleic acids, is characterized in that a single reaction is performed in a sample, but in a state in which the sample is separated into multiple partitions, an individual reaction is performed in each partition. Separation as described above enables measurement sensitive to an amount of nucleic acids. A real-time PCR (qPCR) used in the related art is a method capable of performing quantitative analysis and qualitative analysis of gene expression through a Ct value based on relative quantitative analysis, but has disadvantages in that a standard curve is necessarily required for absolute quantitative analysis and a large deviation in result values may be made depending on efficiency of PCR. Digital PCR developed in order to overcome limitations as described above is a field spotlighted as a new-generation PCR for diagnosis of diseases, and the like, due to advantages that it is possible to analyze a trace amount of a sample such as 10 to 1,000 picoliters of the sample, which is significantly small as compared to real-time PCR, and it is possible to treat a large numbers of samples at the same time. WO2017/209906 discloses an PCR emulsion composition made by mixing (i) a fluid sample containing target DNAs and one or more PCR reagents and (ii) a carrier oil comprising a surfactant, wherein the carrier oil is a mineral oil, a fluorinated oil, a silicone oil, or a combination thereof. D1 also discloses a method for producing the droplet digital PCR emulsion by mixing the fluid sample and the carrier oil.

DNA polymerase is often in an unstable state, and methods of introducing a surfactant or a detergent for stabilization have been suggested. As an example, U.S. patent No. 6,127,155 discloses the fact that a detergent such as polyethoxylated sorbitan monolaurate (Tween 20) and ethoxylated alkyl phenol 4-nonylphenyl-polyethylene glycol (Nonidet P-40) contained in a buffer stabilizes DNA polymerase. However, in some applications, it is known that DNA polymerase stabilized by Tween 20 has low amplification efficiency or amplifies a non-specific product and the detergent such as Nonidet P-40, and the like, is toxic, such that development of a method capable of solving the above-mentioned problems, stabilizing polymerase, and observing a result of a PCR reaction in real-time with high reliability has been required.

### Disclosure of Invention

### Technical Problem

An object of the present invention is to provide a digital polymerase chain reaction (digital PCR) emulsion composition, a preparation method thereof, and a method capable of confirming a PCR reaction in real time with high reliability in a manner different from conventional digital PCR through a method of easily and simply partitioning the emulsion composition.

### Solution to Problem

In one general aspect, a digital PCR emulsion composition for uniformly partitioning a PCR sample includes: an emulsion formed by mixing a PCR sample mixture containing one or two or more amplification target DNAs and a pair of forward and reverse primers for amplifying each amplification target DNA and oil A containing a surfactant with each other; and oil B applied on the outside of the emulsion at a uniform thickness.

The PCR sample mixture may contain one or two or more selected from the group consisting of DNA polymerase, a reaction buffer, dNTP, and a fluorescent material.

The surfactant may have a hydrophilic-lipophilic balance (HLB) value of 4 to 8.

The surfactant may further include a surfactant which has an adjustable HLB value and does not have an influence on an amplification reaction in PCR.

The surfactant may be a non-ionic surfactant.

The oil B may be mineral oil.

The oil A containing the surfactant may be contained in a volume ratio of 1:0.3 to 2 based on the PCR sample mixture.

A particle diameter of the emulsion may be 5 um to 30 um.

In another general aspect, a preparation method of a digital PCR emulsion composition includes: preparing a PCR sample mixture by mixing one or two or more amplification target DNAs and one or more pairs of forward and reverse primers for amplifying each amplification target DNA with each other; and preparing a digital PCR emulsion composition by mixing the PCR sample mixture, oil A containing a surfactant, and oil B with each other.

In the preparing of the PCR sample mixture, the PCR sample mixture may contain one or two or more selected from the group consisting of DNA polymerase, a reaction buffer, dNTP, and a fluorescent material.

The surfactant may have a hydrophilic-lipophilic balance (HLB) value of 4 to 8.

The surfactant may further include a surfactant which has an adjustable HLB value and does not have an influence on an amplification reaction in PCR.

The surfactant may be a non-ionic surfactant.

The oil B may be mineral oil.

The oil A containing the surfactant may be contained in a volume ratio of 1:0.3 to 2 based on the PCR sample mixture.

A particle diameter of the emulsion may be 5 um to 30 um.

In another general aspect, a uniform partitioning method of a PCR sample includes: partitioning the digital PCR emulsion composition described above in a plate to which a reaction film is attached; and rotating the plate to centrifuge and partition the sample.

The plate may be rotated at 1,000 to 20,000 rpm for 10 to 1000 seconds.

A size of lattices of the reaction film may be, for example, 0.6 to 600 times, preferably 2 to 500 times, and more preferably 3.3 to 400 times based on a diameter of emulsion particles.

### Advantageous Effects of Invention

With a digital polymerase chain reaction (digital PCR) emulsion composition for uniformly partitioning a PCR sample, a preparation method thereof, and a uniform partitioning method of a PCR sample according to the present invention, a PCR reagent to be amplified may be easily and rapidly prepared in an emulsion form, and reliability of digital PCR results may be significantly improved by allowing the formed emulsion to be uniformly partitioned using a cylindrical plate to which a reaction film is attached.

### Brief Description of Drawings

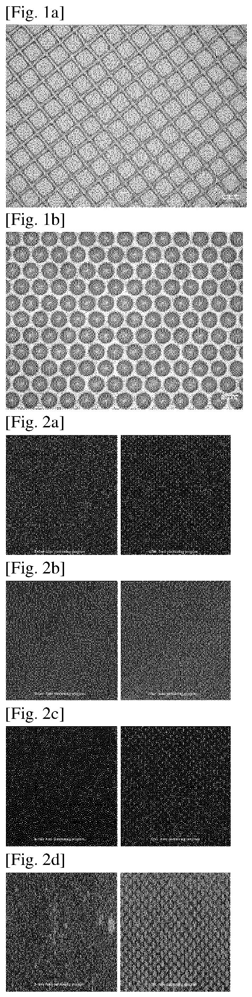

FIGS. 1A and 1B are examples of a reaction film in which an emulsion composition is partitioned.
FIG. 1A shows square wells (100 um) and FIG. 1B shows round wells (100 um).
FIGS. 2A to 2D are examples of partitioning results of emulsion compositions in Examples 1 to 4 using an automatic partitioning program.

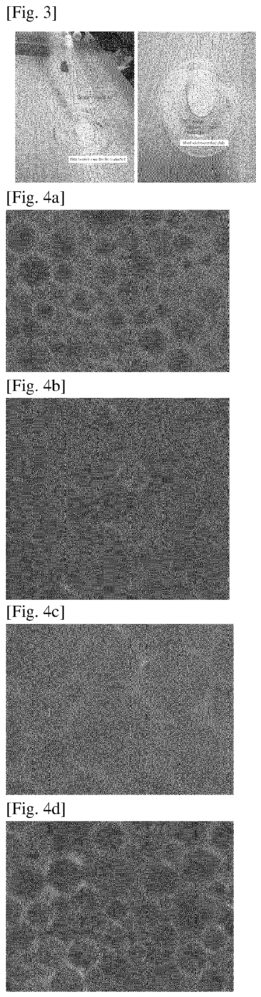

FIG. 3 is an example of partitioning an emulsion composition in a plate to which a reaction film is attached.

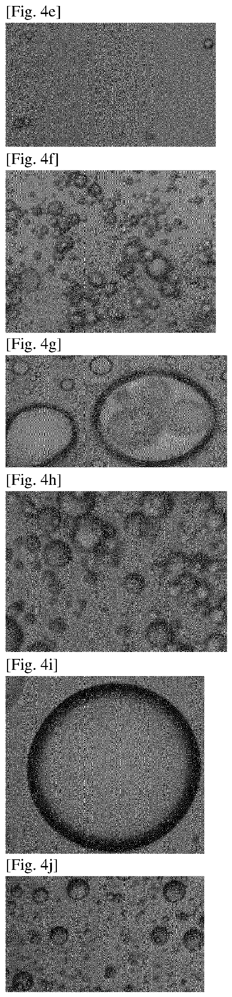

FIGS. 4A to 4J are examples of results of microscope photographs for distribution of emulsion particles formed according to Examples 1 to 4 and Comparative Examples 1 to 6 corresponding to control groups.

### Best Mode for Carrying out the Invention

Hereinafter, a digital PCR emulsion composition for uniformly partitioning a PCR sample according to the present invention will be described in detail with reference to the accompanying tables or drawings.

The drawings to be provided below are provided by way of example. Therefore, the present invention is not limited to the accompanying drawings provided below, but may be modified in many different forms. In addition, the accompanying drawings suggested below will be exaggerated in order to clear the scope of the present invention.

Technical terms and scientific terms used in the present specification have the general meaning understood by those skilled in the art to which the present invention pertains unless otherwise defined, and a description for the known function and configuration unnecessarily obscuring the gist of the present invention will be omitted in the following description and the accompanying drawings.

Further, a singular form used in the specification of the present invention includes a plural form unless particularly described in the text.

Further, a unit used in the specification of the present invention without particular description is based on a weight, and as an example, a unit of % or ratio means a weight % or weight ratio.

Further, in the specification of the present invention, an expression "includes" has an open description having a meaning equivalent to an expression such as "provides", "contains", "has", or "is characterized", and does not exclude elements, materials, or processes that are not additionally mentioned. Further, an expression "is substantially composed of ··" means that another element, material or process that is not mentioned with a specified element, material or process is present in a quantity in which it does not have an unacceptably significant effect on at least one basic and novel technical idea of the invention. In addition, an expression "is composed of" means that only the mentioned element, material, or process is present.

The term "HLB" is well-known to those skilled in the art and indicates hydrophilic-lipophilic balance of a surfactant at 25°C according to Griffin.

The term "hydrophilic-lipophilic balance (HLB)" means balance between a size and strength of a hydrophilic group and a size and strength of a lipophilic group of a surfactant. An HLB value according to Griffin as described above is defined in a document [J. Soc. Cosm. Chem., 1954 (volume 5), pages 249-256].

In the present invention, the term "primer" includes a forward primer and a reverse primer recognizing a sequence of a target gene, and specifically means a primer providing an analysis result having specificity and sensitivity. When the primer is a primer which amplifies only a sequence of a target gene including a complementary primer binding site and does not cause non-specific amplification, the primer may have high specificity.

In the present invention, the term "sample" or "test sample" indicates a target for analysis and used in the sample meaning throughout the specification.

Hereinafter, the digital PCR emulsion composition for uniformly partitioning a PCR sample according to the present invention will be described.

The digital PCR emulsion composition for uniformly partitioning a PCR sample according to the present invention contains: an emulsion formed by mixing a PCR sample mixture containing one or two or more amplification target DNAs and a pair of forward and reverse primers for amplifying each amplification target DNA and oil A containing a surfactant with each other; and oil B applied on the outside of the emulsion at a uniform thickness.

The kind and a size of amplification target DNA are not particularly limited as long as the amplification target DNA is a DNA capable of being amplified by digital PCR, as a pair of primers required for an amplification reaction, any pair of primers may be designed by a primer designing method capable of being easily used by those skilled in the art and used without limitation as long as the primers may recognize the amplification target DNA and amplification can be performed, and in order to improve amplification efficiency, one or more pairs of primers with respect to one kind of amplification target DNA can be mixed and used together, but the present invention not limited thereto.

The oil is not particularly limited as long as it is generally used in the related art, and as a non-restrictive specific example, the oil may be one or more selected from silicone-based oil, hydrocarbon-based oil, ester-based oil, and the like. More specifically, the silicone-based oil may be one or a mixture of two or more selected from cyclopentasiloxane, cyclohexasiloxane, cycloheptasiloxane, cyclomethicone, cy-clophenylmethicone, cyclotetrasiloxane, cyclotrisiloxane, dimethicone, capryldimethicone, caprylyltrimethicone, caprylylmethicone, cetaarylmethicone, hex-adecylmethicone, hexylmethicone, laurylmethicone, myristylmethicone, phenyl-methicone, stearylmethicone, stearyldimethicone, trifluoropropylmethicone, cetyldimethicone, dimethylpolysiloxane, methylphenylpolysiloxane, decamethylcyclopentasiloxane, methyltrimethicone, phenyltrimethicone, and the like, but is not limited thereto. An Example of the hydrocarbon-based oil may include paraffin-based hydrocarbon oil, olefin-based hydrocarbon oil, aliphatic, alicyclic or aromatic hydrocarbon oil, and the like, and as a non-restrictive example thereof, the hydrocarbon-based oil may be one or a mixture of two or more selected from n-octane, n-nonane, n-decane, n-undecane, n-dodecane, n-tridecane, n-tetradecane, n-pentadecane, n-hexadecane, n-heptadecane, n-octadecane, Vaseline, paraffin, isoparaffin, ceresin, squalane, squalene, polybutene, polydecene, polyisoprene, and the like, but is not limited thereto. The ester-based oil may be one or a mixture of two or more selected from ascorbyl palmitate, ascorbyl linoleate, ascorbyl stearate, distearyl maleate, benzyl benzoate, benzyl laurate, butylene glycol dicaprylate/dicaprate, butylene glycol diisononanoate, butylene glycol laurate, butylene glycol stearate, butyl isostearate, cetearyl isononanoate, cetearyl nonanoate, cetyl caprylate, cetylethyl hexanoate, cetyl isononanoate, ethylhexyl caprylate/caprate, ethylhexyl isononanoate, ethylhexyl isostearate, ethylhexyl laurate, hexyl laurate, octyldodecyl isostearate, isopropyl isostearate, isostearyl isononanoate, isostearyl isostearate, isocetylethyl hexanoate, neopentyl glycol dicaprate, neopentyl glycol diethyl hexanoate, neopentyl glycol diisononanoate, neopentyl glycol diisostearate, pentaerythryl stearate, pentaerythryl tetraethyl hexanoate, triethyl hexanoin, and the like, but is not limited thereto.

In another aspect, an oil phase according to an exemplary embodiment of the present invention may be natural oil, synthetic oil, or a mixture thereof. More specifically, as the natural oil, one or a mixture of two or more selected from vegetable oil, animal oil, mineral oil, and the like, may be used. As a non-restrictive specific example, the vegetable oil may be olive oil, almond oil, palm oil, malt oil, castor oil, avocado oil, corn oil, soybean oil, rapeseed oil, camellia oil, cottonseed oil, evening primrose oil, jojoba oil, coconut oil, rosehip oil, macadamia oil, and the like, the animal oil may be egg yolk oil, shark liver oil, emu oil, mink oil, or the like, and the mineral oil may be paraffin, Vaseline, ceresin, but the oil is not limited thereto. The synthetic oil may be the silicon-based oil, the hydrocarbon-based oil, the ester-based oil, or the like, and since a specific component of each synthetic coil is the same as that obtained by excluding natural oil from the above-mentioned silicon-based oil, hydrocarbon-based oil, and ester-based oil, a detailed description thereof will be omitted.

Any kind of surfactant may be used without limitation as long as an object of the present invention may be achieved. As an example, a non-ionic surfactant, a cationic surfactant, an anionic surfactant, or a mixture of one or two or more selected from these surfactants may be appropriately mixed and used, but the surfactant is not limited thereto.

In the digital PCR emulsion composition for uniformly partitioning a PCR sample according to the exemplary embodiment of the present invention, the PCR sample mixture may contain one or two or more selected from the group consisting of DNA polymerase, a reaction buffer, dNTP, and a fluorescent material.

The kind of polymerase is not particularly limited as long as the object of the present invention is achieved. An example of the polymerase may include Taq polymerase, Tth polymerase, Tli or VENT polymerase, Pfu or DEEPVENT polymerase, Pwo polymerase, Bst polymerase, Sac polymerase, Tac polymerase, Tfl/Tub polymerase, Tru polymerase, DYNAZYME polymerase, Tne polymerase, Tma polymerase, Tsp polymerase, Mth polymerase, KOD DNA polymerase, or the like, but is not limited thereto.

The dNPT is a term indicating deoxynucleotide triphosphate, and dNTPs means a mixture of deoxyadenine triphosphate (dATP), deoxycytosine triphosphate (dCTP), de-oxyguanine triphosphate (dGTP), and deoxythymine triphosphate (dTTP).

A PCR premix, which is a reactant prepared in advance before a PCR reaction, means a ready-to-use reagent so that the PCR reaction is carried out after adding the amplification target DNA thereto, and the PCR premix may contain the DNA polymerase, the reaction buffer, the dNTP mixture, and the like, but is not limited thereto.

In the digital PCR emulsion composition for uniformly partitioning a PCR sample according to the exemplary embodiment of the present invention, a hydrophilic-lipophilic balance (HLB) value of the surfactant may be 4 to 8.

In the digital PCR emulsion composition for uniformly partitioning a PCR sample according to the exemplary embodiment of the present invention, the surfactant may further include a surfactant which has an adjustable HLB value and does not have an influence on an amplification reaction in PCR.

In the digital PCR emulsion composition for uniformly partitioning a PCR sample according to the exemplary embodiment of the present invention, the surfactant may be a non-ionic surfactant.

According to the exemplary embodiment of the present invention, the non-ionic surfactant is preferable in that the non-ionic surfactant increases dispersibility to allow the emulsion to be smoothly formed and particles having a size corresponding to a lattice size of the reaction film may be easily formed.

The hydrophilic-lipophilic balance (HLB) value of the non-ionic surfactant is not particularly limited as long as the object of the present invention is achieved, but as a specific example, the HLB value of the non-ionic surfactant is 2 to 10, preferably, 3 to 9, and more preferably 4 to 8, which is effective in that it is possible to decrease occurrence of coagulation between the particles and secure stability of the formed emulsion. In detail, the non-ionic surfactant may be one or more sorbitan fatty acid esters selected from the group consisting of sorbitan monooleate, sorbitan monostearate, sorbitan monopalmitate, sorbitan monolaurate, sorbitan tristearate, sorbitan sesquiolate, and sorbitan trioleate, but is not necessarily limited thereto. This is effective in view of stability of the emulsion, preparation of a gelated molded body, uniformity and sphericity of the molded body, and rapid separation of the molded body prepared in an oil phase.

In the digital PCR emulsion composition for uniformly partitioning a PCR sample according to the exemplary embodiment of the present invention, the oil B may be mineral oil.

The mineral oil may be paraffin, Vaseline, ceresin, or the like, but this is a non-restrictive example of the mineral oil, and the mineral oil is not limited thereto.

In the digital PCR emulsion composition for uniformly partitioning a PCR sample according to the exemplary embodiment of the present invention, as an example, the oil A may be contained at a volume ratio of 0.1 to 4 times, preferably 0.2 to 3 times, and more preferably 0.3 to 2 times based on the PCR sample mixture. Within the above-mentioned range, emulsion particles may be uniformly partitioned in lattices of the reaction film, and there is no interference with a PCR amplification phenomenon depending on the surfactant, such that reliability of digital PCR according to the present invention may be significantly improved. This is a non-restrictive example, but the volume ratio of the oil A is not limited to the above-mentioned numerical range.

In the case in which the PCR sample mixture and the oil A at the above-mentioned volume ratio, it is effective to uniformly form the digital PCR emulsion, which is more effective in view of preparation efficiency and productivity, and PCR sample may be uniformly distributed in the lattices of the reaction film at the time of partitioning. However, this is only a non-restrictive example, but the present invention is not limited to the numerical range described above.

According to the exemplary embodiment of the present invention, as a specific example, a viscosity of the digital PCR emulsion composition is in a range of 100 to 10,000 mPa·S, preferably, 500 to 5,000 mPa·S, but this range is only a non-restrictive example, and the viscosity is not limited thereto. The viscosity is within the above-mentioned range, which is preferable in that it is possible to smoothly form an emulsion having a uniform size from a composition for digital PCR, the emulsion may be partitioned in predetermined lattices on the reaction film attached to an inner wall of the plate, and inference between the lattices (cells) hardly occurs.

In the digital PCR emulsion composition for uniformly partitioning a PCR sample according to the exemplary embodiment of the present invention, a diameter of the emulsion particle may be 1 µm to 60 µm, preferable 3 µm to 45 µm, and more preferably 5µm to 30 µm, and in the case of the emulsion particles having the above-mentioned size, distributed in the oil B, as the size of the emulsion is decreased, the emulsion particle may be partitioned so as to be suitable for the lattice of the reaction film having a small size. However, as long as the object of the present invention is achieved, the lattice of the reaction film and the diameter of the emulsion particles are not limited to the above-mentioned range.

Hereinafter, a preparation method of a digital PCR emulsion composition for uniformly partitioning a PCR sample according to the present invention will be described in detail.

The preparation method of a digital PCR emulsion composition for uniformly partitioning a PCR sample includes: preparing a PCR sample mixture by mixing one or two or more amplification target DNAs and one or more pairs of forward and reverse primers for amplifying each amplification target DNA with each other; and preparing a digital PCR emulsion composition by mixing the PCR sample mixture, oil A containing a surfactant, and oil B with each other.

The kind and a size of amplification target DNA are not particularly limited as long as the amplification target DNA is a DNA capable of being amplified by digital PCR, as a pair of primers required for an amplification reaction, any pair of primers may be designed by a primer designing method capable of being easily used by those skilled in the art and then used without limitation as long as the primers may recognize the amplification target DNA and amplification can be performed, and in order to improve amplification efficiency, one or more pairs of primers with respect to one kind of amplification target DNA can be mixed and used together, but the present invention is not limited thereto.

The oil is not particularly limited as long as it is generally used in the related art, and as a non-restrictive specific example, the oil may be one or more selected from silicone-based oil, hydrocarbon-based oil, ester-based oil, and the like. More specifically, the silicone-based oil may be one or a mixture of two or more selected from cyclopentasiloxane, cyclohexasiloxane, cycloheptasiloxane, cyclomethicone, cy-clophenylmethicone, cyclotetrasiloxane, cyclotrisiloxane, dimethicone, capryldimethicone, caprylyltrimethicone, caprylylmethicone, cetaarylmethicone, hex-adecylmethicone, hexylmethicone, laurylmethicone, myristylmethicone, phenyl-methicone, stearylmethicone, stearyldimethicone, trifluoropropylmethicone, cetyldimethicone, dimethylpolysiloxane, methylphenylpolysiloxane, decamethylcyclopentasiloxane, methyltrimethicone, phenyltrimethicone, and the like, but is not limited thereto. An Example of the hydrocarbon-based oil may include paraffin-based hydrocarbon oil, olefin-based hydrocarbon oil, aliphatic, alicyclic or aromatic hydrocarbon oil, and the like, and a non-restrictive example thereof may be one or a mixture of two or more selected from n-octane, n-nonane, n-decane, n-undecane, n-dodecane, n-tridecane, n-tetradecane, n-pentadecane, n-hexadecane, n-heptadecane, n-octadecane, Vaseline, paraffin, isoparaffin, ceresin, squalane, squalene, polybutene, polydecene, polyisoprene, and the like, but is not limited thereto. The ester-based oil may be one or a mixture of two or more selected from ascorbyl palmitate, ascorbyl linoleate, ascorbyl stearate, distearyl maleate, benzyl benzoate, benzyl laurate, butylene glycol dicaprylate/dicaprate, butylene glycol diisononanoate, butylene glycol laurate, butylene glycol stearate, butyl isostearate, cetearyl isononanoate, cetearyl nonanoate, cetyl caprylate, cetylethyl hexanoate, cetyl isononanoate, ethylhexyl caprylate/caprate, ethylhexyl isononanoate, ethylhexyl isostearate, ethylhexyl laurate, hexyl laurate, octyldodecyl isostearate, isopropyl isostearate, isostearyl isononanoate, isostearyl isostearate, isocetylethyl hexanoate, neopentyl glycol dicaprate, neopentyl glycol diethyl hexanoate, neopentyl glycol diisononanoate, neopentyl glycol diisostearate, pentaerythryl stearate, pentaerythryl tetraethyl hexanoate, triethyl hexanoin, and the like, but is not limited thereto.

In another aspect, an oil phase according to an exemplary embodiment of the present invention may be natural oil, synthetic oil, or a mixture thereof. More specifically, as the natural oil, one or a mixture of two or more selected from vegetable oil, animal oil, mineral oil, and the like, may be used. As a non-restrictive specific example, the vegetable oil may be olive oil, almond oil, palm oil, malt oil, castor oil, avocado oil, corn oil, soybean oil, rapeseed oil, camellia oil, cottonseed oil, evening primrose oil, jojoba oil, coconut oil, rosehip oil, macadamia oil, and the like, the animal oil may be egg yolk oil, shark liver oil, emu oil, mink oil, or the like, and the mineral oil may be paraffin, Vaseline, ceresin, but the oil is not limited thereto. The synthetic oil may be the silicon-based oil, the hydrocarbon-based oil, the ester-based oil, or the like, and since a specific component of each synthetic coil is the same as that obtained by excluding natural oil from the above-mentioned silicon-based oil, hydrocarbon-based oil, and ester-based oil, a detailed description thereof will be omitted.

Any kind of surfactant may be used without limitation as long as an object of the present invention may be achieved. As an example, a non-ionic surfactant, a cationic surfactant, an anionic surfactant, or a mixture of one or two or more selected from these surfactants may be appropriately mixed and used, but the surfactant is not limited thereto.

A content ratio of the oil A containing the surfactant and the oil B is not limited as long as the object of the present invention is achieved, but as a specific example, the oil B may be contained at a volume ratio of 0.1 to 100, preferably 1 to 50, and more preferably 10 to 30 based on the oil A. The oil A and the oil B are mixed at the above-mentioned ratio, which is preferable in that the emulsion may be effectively formed, and stability is improved.

In the preparation method of a digital PCR emulsion composition for uniformly partitioning a PCR sample according to the exemplary embodiment of the present invention, the PCR sample mixture may contain one or two or more selected from the group consisting of DNA polymerase, a reaction buffer, dNTP, and a fluorescent material.

The kind of polymerase is not particularly limited as long as the object of the present invention is achieved. An example of the polymerase may include Taq polymerase, Tth polymerase, Tli or VENT polymerase, Pfu or DEEPVENT polymerase, Pwo polymerase, Bst polymerase, Sac polymerase, Tac polymerase, Tfl/Tub polymerase, Tru polymerase, DYNAZYME polymerase, Tne polymerase, Tma polymerase, Tsp polymerase, Mth polymerase, KOD DNA polymerase, or the like, but is not limited thereto.

The dNPT is a term indicating deoxynucleotide triphosphate, and dNTPs means a mixture of deoxyadenine triphosphate (dATP), deoxycytosine triphosphate (dCTP), de-oxyguanine triphosphate (dGTP), and deoxythymine triphosphate (dTTP).

A PCR premix, which is a reactant prepared in advance before a PCR reaction, means a ready-to-use reagent so that the PCR reaction is carried out after adding the amplification target DNA thereto, and the PCR premix may contain the DNA polymerase, the reaction buffer, the dNTP mixture, and the like, but is not limited thereto.

In the preparation method of a digital PCR emulsion composition for uniformly partitioning a PCR sample according to the exemplary embodiment of the present invention, a hydrophilic-lipophilic balance (HLB) value of the surfactant may be 4 to 8.

In the preparation method of a digital PCR emulsion composition for uniformly partitioning a PCR sample according to the exemplary embodiment of the present invention, the surfactant may further include a surfactant of which an HLB value may be adjusted and which does not have an influence on an amplification reaction in PCR.

In the preparation method of a digital PCR emulsion composition for uniformly partitioning a PCR sample according to the exemplary embodiment of the present invention, the surfactant may be a non-ionic surfactant.

According to the exemplary embodiment of the present invention, the non-ionic surfactant is preferable in that the non-ionic surfactant increases dispersibility to allow the emulsion to be smoothly formed and particles having a size corresponding to a lattice size of the reaction film may be easily formed.

The hydrophilic-lipophilic balance (HLB) value of the non-ionic surfactant is not particularly limited as long as the object of the present invention is achieved, but as a specific example, the HLB value of the non-ionic surfactant is 2 to 10, preferably, 3 to 9, and more preferably 4 to 8, which is effective in that it is possible to decrease occurrence of coagulation between the particles and secure stability of the formed emulsion. In detail, the non-ionic surfactant may be one or more sorbitan fatty acid esters selected from the group consisting of sorbitan monooleate, sorbitan monostearate, sorbitan monopalmitate, sorbitan monolaurate, sorbitan tristearate, sorbitan sesquiolate, and sorbitan trioleate, but is not necessarily limited thereto. This is effective in view of stability of the emulsion, preparation of a gelated molded body, uniformity and sphericity of the molded body, and rapid separation of the molded body prepared in an oil phase.

In the preparation method of a digital PCR emulsion composition for uniformly partitioning a PCR sample according to the exemplary embodiment of the present invention, the oil B may be mineral oil.

The mineral oil may be paraffin, Vaseline, ceresin, or the like, but this is a non-restrictive example of the mineral oil, and the mineral oil is not limited thereto.

In the preparation method of a digital PCR emulsion composition for uniformly partitioning a PCR sample according to the exemplary embodiment of the present invention, as an example, the oil A may be contained at a volume ratio of 0.1 to 4 times, preferably 0.2 to 3 times, and more preferably 0.3 to 2 times based on the PCR sample mixture. Within the above-mentioned range, emulsion particles may be uniformly partitioned in lattices of the reaction film, and there is no interference with a PCR amplification phenomenon depending on the surfactant, such that reliability of digital PCR according to the present invention may be significantly improved. This is a non-restrictive example, but a content of the oil A is not limited to the above-mentioned numerical range.

In the case in which the PCR sample mixture and the oil A at the above-mentioned volume ratio, it is effective to uniformly form the digital PCR emulsion, which is more effective in view of preparation efficiency and productivity, and PCR sample may be uniformly distributed in the lattices of the reaction film at the time of partitioning. However, this is only a non-restrictive example, but the present invention is not limited to the numerical range described above.

A content ratio of the oil A containing the surfactant and the oil B is not limited as long as the object of the present invention is achieved, but as a specific example, the oil B may be contained at a volume ratio of 0.1 to 100, preferably 1 to 50, and more preferably 10 to 30 based on the oil A. The oil A and the oil B are mixed at the above-mentioned ratio, which is preferable in that the emulsion may be effectively formed, and stability is improved.

In the preparation method of a digital PCR emulsion composition for uniformly partitioning a PCR sample according to the exemplary embodiment of the present invention, a diameter of the emulsion particle may be 1 µm to 60 µm, preferably 3 µm to 45 µm, and more preferably 5 to 30 µm, and in the case of the emulsion particles having the above-mentioned size, distributed in the oil B, as the size of the emulsion is decreased, the emulsion particle may be partitioned so as to be suitable for the lattice of the reaction film having a small size. However, as long as the object of the present invention is achieved, the lattice of the reaction film and the diameter of the emulsion particles are not limited to the above-mentioned range.

Hereinafter, a uniform partitioning method of a PCR sample according to the present invention will be described in detail.

The uniform partitioning method of a PCR sample according to the present invention includes: partitioning a digital PCR emulsion composition for uniformly partitioning the PCR sample in a plate to which a reaction film is attached; and rotating the plate to centrifuge and partition the sample.

A material, a size, and the like of the plate are not particularly limited as long as the plate has a cylindrical shape and a storage place so that the emulsion composition may be injected thereinto and stored therein. The digital PCR emulsion composition according to the present invention injected into the plate is centrifuged by rotation of the plate to thereby uniformly partitioned in each cell of the reaction film attached to the inside of the plate and having a lattice pattern shape. In this way, it is possible to confirm progress of a reaction of the PCR sample by a temperature changed with the passage of time in a PCR amplification process in real-time, and it is possible to maintain the PCR sample distributed in a lattice shaped cell so as to be mixed with a cell adjacent thereto, such that reliability of a digital PCR result may be significantly improved.

In the uniform partitioning method of a PCR sample according to the exemplary embodiment of the present invention, as an example, the plate may be rotated at 1,000 to 20,000 rpm, preferably 1,500 to 15,000 rpm, and more preferably, 2,000 to 10,000 rpm for 10 to 1,000 seconds, preferably 50 to 750 seconds, and more preferably 100 to 500 seconds.

In the case in which the above-mentioned ranges are satisfied, the formed emulsion may be more uniformly distributed on the reaction film, but this case is only a non-restrictive example, and the present invention is not limited to the above-mentioned numerical ranges.

In the uniform partitioning method of a PCR sample according to the exemplary embodiment of the present invention, as an example, a size of the lattice of the reaction film may be 0.5 to 600 times, preferably 2 to 500 times, and more preferably 3.3 to 400 times the diameter of the emulsion particle, but this is only an example, and the size of the lattice is not limited to the above-mentioned numerical range.

In the uniform partitioning method of a PCR sample according to the exemplary embodiment of the present invention, the size of the lattice of the reaction film may be 100 um to 2 mm, and the diameter of the emulsion particle may be 5 um to 30 um, and in the case of the emulsion particle distributed in the oil B, the size of the emulsion particle is decreased, the emulsion particle may be partitioned in a lattice having a small size. However, this does not mean that it is impossible to partition an emulsion particle having a small diameter in a large film lattice, and this corresponds to a relationship for injecting an emulsion particle having a small diameter in a film lattice having a significantly small size. However, as long as the object of the present invention is achieved, the lattice of the reaction film and the diameter of the emulsion particles are not limited to the above-mentioned range.

The size of the lattice of the reaction film and the diameter of the emulsion particle are out of the ranges according to the present invention, which may act as a factor inhibiting a PCR amplification reaction, and in order to prevent this phenomenon, an emulsion composition having a diameter satisfying the size of the lattice of the reaction film may be added.

In the uniform partitioning method of a PCR sample according to the exemplary embodiment of the present invention, a content of the PCR reagent, and the oil A containing the surfactant may have a large influence on the size of the emulsion particles, and as a specific example, a mixing volume ratio of the PCR reagent and the oil A containing the surfactant may be 1: 0.1 to 4, preferably 1: 0.2 to 3, and more preferably, 1: 0.3 to 2. Within the above-mentioned range, the emulsion particles may be uniformly partitioned in the lattice of the reaction film, and interference with the PCR amplification phenomenon depending on the surfactant may not be generated, such that reliability of digital PCR according to the present invention may be significantly improved. However, this is only a non-restrictive example, but the present invention is not limited to the above-mentioned numerical range.

Hereinafter, the contents of the present invention will be described in more detail through Examples. Examples are to describe the present invention in detail, and the scope of the present invention is not limited thereto.

### [Experimental Materials, Reagent, and Equipment]

- DNA premix: mixture of DNA polymerase, reaction buffer, dNTP, and the like (Bioneer, Korea)
- Fluorescent material (dye): tetrachlorofluorescein (Bioneer, Korea)
- amplification target DNA: *csf2* (Bioneer, Korea)
- Forward primer: 5'-catctcagaaatgtttgacctccag-3' (25mer)
- Reverse primer: 5'-cactacaagcagcactgccctc-3' (22mer)
- Surfactant having an HLB value of 4 to 8: Span 20 (Sigma-Aldrich)
- Tween 80 (Sigma-Aldrich)
- Triton X-100 (Sigma-Aldrich)
- Oil B: mineral oil M5904 (Sigma-Aldrich)
- sdPCR Equipment: sdPCR equipment manufactured by RevoSketch and disclosed in International Patent No. WO2018/194240 A1

### [Preparation Example]

### 1. Preparation of PCR Sample Mixture

The DNA premix (Bioneer, Korea), the amplification target DNA, a pair of forward and reverse primers, the fluorescent material (dye), and distilled water were mixed in a 15 mL conical tube at a mixing ratio shown in the following Table 1, thereby preparing a PCR sample mixture.

**[Table 1]**

| Kind | Content (uL) |
|---|---|
| DNA Premix | **25.0** |
| Amplification Target DNA | **1.0** |
| Forward Primer | **1.0** |
| Reverse Primer | **1.0** |
| Fluorescent Material (Dye) | **1.0** |
| Distilled Water | **21.0** |

### 2. Preparation of Oil A Containing Surfactant

In a tube, 450 uL of surfactant (Span20; HLB value: 6, Sigma-Aldrich, U.S.), 5 uL of Triton X-100, 45 uL of Tween80, and 9,500 uL of mineral oil M5904 were mixed with each other, thereby preparing oil A containing a total of 10,000 uL of surfactant.

### 3. Preparation of Emulsion Composition

Ingredients according to respective Examples and Comparative Examples shown in the following Table 2 were mixed in respective tubes so as to each have a volume of 2,000 uL, and subjected to vortexing for 30 seconds to thereby be emulsified.

Here, in Example 1 and Comparative Examples 1 to 6, a reaction film having a lattice size of 2,000 um was used.

**[Table 2]**

| Ingredients of Emulsion Composition (Vortexing time: 30 seconds) | | | | | |
|---|---|---|---|---|---|
| | PCR Sample Mixture (uL) | Oil A (uL) Containing Surfactant | Oil B (Mineral Oil M5904 ) (uL) | Distilled Water (uL) | Lattice Size of Reaction Film(Diameter of Emulsion Particle) (um) |
| Example 1 | 150 | 100 | 1,600 | 150 | **2,000(about 10)** |
| Example 2 | 50 | 50 | 1,800 | 100 | **1,000(5 to 30)** |
| Example 3 | 50 | 50 | 1,900 | 0 | **100(10 to 30)** |
| Example 4 | 50 | 100 | 1,850 | 0 | **100(5 to 15)** |
| Comparative Example 1 | 150 | 0 | 1,700 | 150 | **2,000(Emulsion was not formed)** |
| Comparative Example 2 | 150 | 1700 | 0 | 150 | **2,000(An amount of the surfactant was significantly large, such that a PCR reaction may have been inhibited.)** |
| Comparative Example 3 | 150 | 10 | 1,690 | 150 | **2,000(Emulsion was not formed)** |
| Comparative Example 4 | 150 | 900 | 800 | 150 | **2,000(An amount of the surfactant was significantly large, such that a PCR reaction may have been inhibited.)** |
| Comparative Example 5 | 50 | 1 | 1,799 | 150 | **2,000(Emulsion was not formed)** |
| Comparative Example 6 | 50 | 300 | 1,500 | 150 | **2,000(An amount of the surfactant was significantly large, such that a PCR reaction may have been inhibited)** |

Mineral oil M5904 was additionally added to each tube in which the ingredients shown in Table 2 were emulsified so as to have a total volume of 10,000 uL.

Thereafter, in order to allow the emulsified solution and the mineral oil M5904 to be well-mixed, vortexing was performed only in a degree in which the emulsified solution and the mineral oil M5904 were mixed with each other, thereby preparing each digital PCR emulsion composition.

As a result of preparing the digital PCR emulsion composition using the ingredients according to the present invention, it was confirmed that the PCR sample was partitioned so as to fit within the lattice having a predetermined size, and as a result of preparing a composition using ingredients out of the content range of the present invention, it was confirmed that formation of the emulsion was incomplete, or the PCR sample was mixed over the lattice (cell) due to interference between emulsions, thereby significantly decreasing reliability of digital PCR, or an excessively large amount of the surfactant was contained to directly inhibit a PCR reaction, thereby decreasing PCR efficiency and significantly decreasing reliability of digital PCR.

### [Experimental Example 1] Partitioning of Emulsion Solution

Among the emulsion compositions prepared in Preparation Examples, the emulsion composition of Example 1 was partitioned in each cylindrical plate to which a reaction film was attached, and mounted in sdPCR equipment manufactured by RevoSketch (disclosed in International Patent No. WO2018/194240 A1) to which an automatic partitioning program was applied, such that the emulsion was centrifuged under the conditions shown in the following Table 3.

**[Table 3]**

| | Centrifugation Rotation Speed (rpm) | Centrifugation Time (Seconds) |
|---|---|---|
| Example 5 | 5,800 | 300 |
| Example 6 | 2,400 | 150 |
| Example 7 | 4,800 | 300 |
| Example 8 | 5,800 | 150 |

Thereafter, fluorescent of the lattice of the reaction film was measured, thereby observing a partitioning result of the emulsion.

The results are shown in FIGS. 2A to 2D.

As a result of applying the sdPCR automatic partitioning program manufactured by RevoSketch, it may be confirmed that the emulsion particles were uniformly partitioned in a cell of each lattice as shown in FIGS. 2A to 2D, and the emulsion was partitioned in a state in which interference between adjacent cells were hardly generated. Therefore, the PCR reaction occurred in each cell may be confirmed in real-time with the passage of time, and thus, the present inventors confirmed that reliability of the PCR reaction was significantly improved, thereby completing the present invention.

Hereinabove, although the present invention has been described by specific matters, exemplary embodiments, and drawings, they have been provided only for assisting in the entire understanding of the present invention. Therefore, the present invention is not limited to the exemplary embodiments. Various modifications and changes may be made by those skilled in the art to which the present invention pertains from this description.

## Claims

1. A digital PCR emulsion composition for uniformly partitioning a PCR sample, the digital PCR emulsion composition comprising:
an emulsion formed by mixing a PCR sample mixture containing one or two or more amplification target DNAs and a pair of forward and reverse primers for amplifying each amplification target DNA and oil A containing a surfactant with each other; and
oil B applied on the outside of the emulsion at a uniform thickness,
wherein the surfactant has a hydrophilic-lipophilic balance (HLB) value of 4 to 8.

2. The digital PCR emulsion composition of claim 1, wherein the PCR sample mixture is two or more selected from the group consisting of DNA polymerase, a reaction buffer, dNTP, and a fluorescent material.

3. The digital PCR emulsion composition of claim 1, wherein the surfactant further includes a surfactant which has an adjustable HLB value and does not have an influence on an amplification reaction in PCR.

4. The digital PCR emulsion composition of claim 1, wherein the surfactant is a non-ionic surfactant.

5. The digital PCR emulsion composition of claim 1, wherein the oil B is mineral oil.

6. The digital PCR emulsion composition of claim 1, wherein the oil A containing the surfactant is contained in a volume ratio of 1:0.3 to 1:2 based on the PCR sample mixture.

7. The digital PCR emulsion composition of claim 1, wherein a particle size of the emulsion is 5 µm to 30 µm.

8. A preparation method of a digital PCR emulsion composition for uniformly partitioning a PCR sample, the preparation method comprising:
preparing a PCR sample mixture by mixing one or two or more amplification target DNAs and one or more pairs of forward and reverse primers for amplifying each amplification target DNA with each other; and
preparing a digital PCR emulsion composition by mixing the PCR sample mixture, oil A containing a surfactant, and oil B with each other,
wherein the surfactant has a hydrophilic-lipophilic balance (HLB) value of 4 to 8.

9. The preparation method of claim 8, wherein in the preparing of the PCR sample mixture, the PCR sample mixture contains two or more selected from the group consisting of DNA polymerase, a reaction buffer, dNTP, and a fluorescent material.

10. The preparation method of claim 8, wherein the surfactant further includes a surfactant which has an adjustable **HLB** value and does not have an influence on an amplification reaction in PCR.

11. A uniform partitioning method of a PCR sample, the uniform partitioning method comprising:
partitioning the digital PCR emulsion composition of any one of claims 1 to 7 in a plate to which a reaction film is attached; and
rotating the plate to centrifuge and partition the sample,
wherein the reaction film is cells with square wells or round wells attached to the inside of the plate.

12. The uniform partitioning method of claim 11, wherein the plate is rotated at 1,000 to 20,000 rpm for 10 to 1000 seconds.

13. The uniform partitioning method of claim 11, wherein a size of cells of the reaction film is 3.3 to 400 times based on a diameter of emulsion particles.

## Patentansprüche

1. Digitale PCR-Emulsionszusammensetzung zum gleichmäßigen Aufteilen einer PCR-Probe, wobei die digitale PCR-Emulsionszusammensetzung umfasst:
eine Emulsion, die durch Mischen einer PCR-Probenmischung, die eine oder zwei oder mehr Amplifikations-Ziel-DNAs und ein Paar von Vorwärts- und Rückwärtsprimern zum Amplifizieren jeder Amplifikations-Ziel-DNA enthält, und Öl A, das ein Tensid enthält, miteinander gebildet wird; und
Öl B, das in gleichmäßiger Dicke auf die Außenseite der Emulsion aufgetragen wird,
wobei das Tensid einen HLB-Wert (Hydrophilic-Lipophilic Balance) von 4 bis 8 aufweist.

2. Digitale PCR-Emulsionszusammensetzung nach Anspruch 1, wobei die PCR-Probenmischung zwei oder mehr ist, ausgewählt aus der Gruppe bestehend aus DNA-Polymerase, einem Reaktionspuffer, dNTP und einem fluoreszierenden Material.

3. Digitale PCR-Emulsionszusammensetzung nach Anspruch 1, wobei das Tensid ferner ein Tensid umfasst, das einen einstellbaren HLB-Wert aufweist und keinen Einfluss auf eine Amplifikationsreaktion in der PCR hat.

4. Digitale PCR-Emulsionszusammensetzung nach Anspruch 1, wobei das Tensid ein nichtionisches Tensid ist.

5. Digitale PCR-Emulsionszusammensetzung nach Anspruch 1, wobei das Öl B Mineralöl ist.

6. Digitale PCR-Emulsionszusammensetzung nach Anspruch 1, wobei das Öl A, das das Tensid enthält, in einem Volumenverhältnis von 1:0,3 bis 1:2, bezogen auf die PCR-Probenmischung, enthalten ist.

7. Digitale PCR-Emulsionszusammensetzung nach Anspruch 1, wobei die Partikelgröße der Emulsion 5 µm bis 30 µm beträgt.

8. Verfahren zur Herstellung einer digitalen PCR-Emulsionszusammensetzung zum gleichmäßigen Aufteilen einer PCR-Probe, wobei das Herstellungsverfahren umfasst:
Herstellen einer PCR-Probenmischung durch Mischen einer oder zweier oder mehrerer Amplifikations-Ziel-DNAs und eines oder mehrerer Paare von Vorwärts- und Rückwärtsprimern zum Amplifizieren jeder Amplifikations-Ziel-DNA miteinander; und
Herstellen einer digitalen PCR-Emulsionszusammensetzung durch Mischen der PCR-Probenmischung, Öl A, das ein Tensid enthält, und Öl B miteinander,
wobei das Tensid einen HLB-Wert (Hydrophilic-Lipophilic Balance) von 4 bis 8 aufweist.

9. Herstellungsverfahren nach Anspruch 8, wobei bei der Herstellung der PCR-Probenmischung die PCR-Probenmischung zwei oder mehr aus der Gruppe bestehend aus DNA-Polymerase, einem Reaktionspuffer, dNTP und einem fluoreszierenden Material enthält.

10. Herstellungsverfahren nach Anspruch 8, wobei das Tensid ferner ein Tensid umfasst, das einen einstellbaren HLB-Wert aufweist und keinen Einfluss auf eine Amplifikationsreaktion in der PCR hat.

11. Verfahren zur gleichmäßigen Aufteilung einer PCR-Probe, wobei das Verfahren zur gleichmäßigen Aufteilung umfasst:
Aufteilen der digitalen PCR-Emulsionszusammensetzung nach einem der Ansprüche 1 bis 7 in einer Platte, an der ein Reaktionsfilm angebracht ist; und
Rotation der Platte, um die Probe zu zentrifugieren und aufzuteilen,
wobei der Reaktionsfilm Zellen mit quadratischen oder runden Vertiefungen darstellt, die an der Innenseite der Platte angebracht sind.

12. Gleichmäßiges Aufteilungsverfahren nach Anspruch 11, wobei die Platte 10 bis 1000 Sekunden lang mit 1000 bis 20000 U/min rotieren gelassen wird.

13. Gleichmäßiges Aufteilungsverfahren nach Anspruch 11, wobei die Größe der Zellen des Reaktionsfilms das 3,3-bis 400-fache des Durchmessers der Emulsionspartikel beträgt.

## Revendications

1. Composition d'émulsion pour PCR numérique destinée à partitionner uniformément un échantillon de PCR, la composition d'émulsion pour PCR numérique comprenant :
une émulsion formée en mélangeant entre eux un mélange d'échantillons de PCR contenant un ou deux ou plusieurs ADN cibles d'amplification et une paire d'amorces sens et antisens pour amplifier chaque ADN cible d'amplification, et une huile A contenant un tensioactif ; et
une huile B appliquée à l'extérieur de l'émulsion en une épaisseur uniforme,
dans laquelle le tensioactif a une valeur d'équilibre hydrophile-lipophile (HLB) comprise entre 4 et 8.

2. Composition d'émulsion pour PCR numérique selon la revendication 1, dans laquelle le mélange d'échantillons de PCR est deux ou plusieurs éléments choisis parmi le groupe constitué d'une ADN polymérase, d'un tampon de réaction, de dNTP et d'un matériau fluorescent.

3. Composition d'émulsion pour PCR numérique selon la revendication 1, dans laquelle le tensioactif comprend en outre un tensioactif qui a une valeur HLB ajustable et n'a pas d'influence sur une réaction d'amplification dans la PCR.

4. Composition d'émulsion pour PCR numérique selon la revendication 1, dans laquelle le tensioactif est un tensioactif non ionique.

5. Composition d'émulsion pour PCR numérique selon la revendication 1, dans laquelle l'huile B est une huile minérale.

6. Composition d'émulsion pour PCR numérique selon la revendication 1, dans laquelle l'huile A contenant le tensioactif est contenue dans un rapport volumique de 1:0,3 à 1:2 par rapport au mélange d'échantillons PCR.

7. Composition d'émulsion pour PCR numérique selon la revendication 1, dans laquelle la taille des particules de l'émulsion est comprise entre 5 µm et 30 µm.

8. Procédé de préparation d'une composition d'émulsion pour PCR numérique destinée à partitionner uniformément un échantillon PCR, le procédé de préparation comprenant :
la préparation d'un mélange d'échantillons PCR en mélangeant un ou deux ou plusieurs ADN cibles d'amplification et une ou plusieurs paires d'amorces sens et antisens pour amplifier chaque ADN cible d'amplification les uns avec les autres ; et
la préparation d'une composition d'émulsion pour PCR numérique en mélangeant entre eux le mélange d'échantillons PCR, l'huile A contenant un tensioactif et l'huile B,
dans laquelle le tensioactif a une valeur d'équilibre hydrophile-lipophile (HLB) comprise entre 4 et 8.

9. Procédé de préparation selon la revendication 8, dans lequel, lors de la préparation du mélange d'échantillons PCR, le mélange d'échantillons PCR contient deux ou plusieurs éléments choisis parmi le groupe comprenant une ADN polymérase, un tampon de réaction, du dNTP et un matériau fluorescent.

10. Procédé de préparation selon la revendication 8, dans lequel le tensioactif comprend en outre un tensioactif qui a une valeur HLB ajustable et qui n'a pas d'influence sur une réaction d'amplification dans la PCR.

11. Procédé de partitionnement uniforme d'un échantillon PCR, le procédé de partitionnement uniforme comprenant :
le partitionnement de la composition d'émulsion PCR numérique de l'une quelconque des revendications 1 à 7 dans une plaque à laquelle est fixé un film de réaction ; et
la rotation de la plaque pour centrifuger et partitionner l'échantillon,
dans lequel le film de réaction est constitué de cellules avec des puits carrés ou ronds fixés à l'intérieur de la plaque.

12. Procédé de partitionnement uniforme selon la revendication 11, dans lequel la plaque est mise en rotation à une vitesse comprise entre 1 000 et 20 000 tr/min pendant 10 à 1 000 secondes.

13. Procédé de partitionnement uniforme selon la revendication 11, dans lequel la taille des cellules du film de réaction est comprise entre 3,3 et 400 fois le diamètre des particules d'émulsion.
